# EUROPEAN PATENT APPLICATION

(11) **EP 4 520 381 A1**
(43) Date of publication of application: **12.03.2025**
(21) Application number: 23816137.6
(22) Date of filing: 01.06.2023
(51) Int. Cl.: A61M 31/00, A61M 37/00, A61F 2/16, A61B 17/00, C12M 1/00

(54) **TRANSFER INSTRUMENT**

(30) Priority: 01.06.2022 JP 2022089707
(71) Applicant: Terumo Kabushiki Kaisha, Tokyo 151-0072 (JP)
(72) Inventor: HAYAKAWA, Koichi, Ashigarakami-gun, Kanagawa 259-0151 (JP)
(74) Representative: Prüfer & Partner mbB Patentanwälte · Rechtsanwälte
(86) International application number: PCT/JP2023/020396
(87) International publication number: WO 2023/234380

(57) **Abstract**

A transfer instrument (10) transfers a medical sheet (300) to a recipient site (402) of a heart (400). The transfer instrument (10) includes a first carrier member (18) including a first shaft (24) and a first support portion (26), and a second carrier member (20) including a movement portion (48) and a second support portion (50), the movement portion (48) including a second shaft (54). The second carrier member (20) is movable relative to the first carrier member (18) from a first position where the second support surface (74) on which the medical sheet (300) is placed in entirely positioned on a first support surface (46) to a second position where the second support surface (74) is positioned in a distal end direction relative to a distal end of the first support portion (26).

## Description

### Technical Field

The present invention relates to a transfer instrument.

### Background Art

JP 2009-511 A discloses a transfer instrument for transferring a medical sheet (cell sheet) for use in, for example, organ transplantation to a treatment site of a living body. The transfer instrument includes a shaft and a support portion provided at a distal portion of the shaft. The support portion includes a support surface on which the medical sheet is placed. The transfer instrument is slid with an upper surface of the medical sheet placed on the support surface pressed with forceps or the like to transfer the medical sheet from the support portion to the treatment site.

### Summary of Invention

The above-described related art requires that, when transferring the medical sheet from the support portion to the treatment site of the living body, the transfer instrument be manipulated with the upper surface of the medical sheet pressed with forceps or the like, so that there is possibility that the medical sheet cannot be efficiently transferred to the treatment site.

It is therefore an object of the present invention to solve the above-described problem.
(1) An aspect of the present invention is a transfer instrument that transfers a medical sheet to a treatment site of a living body, the transfer instrument including: a first carrier member including a first shaft and a first support portion, the first support portion being provided at a distal portion of the first shaft and including a first support surface; and a second carrier member including a movement portion and a second support portion, the movement portion including a second shaft extending along an axial direction of the first shaft, the second support portion being provided at a distal portion of the movement portion and including a second support surface smaller than the first support surface, in which the first support surface supports an overhanging portion of the medical sheet, the overhanging portion sticking out over the second support surface with the second support portion positioned on the first support surface, and the second carrier member is movable relative to the first carrier member from a first position where the second support surface is entirely positioned on the first support surface to a second position where the second support surface is positioned in a distal end direction relative to a distal end of the first support portion.

According to the present invention, the medical sheet is supported by the first support surface and the second support surface with the second carrier member at the first position. It is therefore possible to prevent the overhanging portion of the medical sheet from becoming crinkled before the medical sheet is transferred to the treatment site of the living body.

It is further possible to move, by moving the second carrier member from the first position to the second position, the second support portion from the first support portion onto the treatment site of the living body to place the overhanging portion of the medical sheet on the treatment site. Subsequently, it is possible to bring, by withdrawing the second support portion from between the medical sheet and the treatment site, the whole of the medical sheet into contact with the treatment site. It is therefore possible to efficiently transfer the medical sheet to the treatment site.

(2) In the transfer instrument according to the above (1), the first shaft may be formed in a tubular shape, and the second shaft may be inserted into a lumen of the first shaft so as to be movable along the axial direction of the first shaft.

(3) In the transfer instrument according to the above (1) or (2), the second support portion may be formed in a sheet shape.

(4) In the transfer instrument according to any one of the above (1) to (3), the second support surface may be entirely positioned in the distal end direction relative to the distal end of the first support portion with the second carrier member at the second position.

(5) In the transfer instrument according to any one of the above (1) to (4), the movement portion may include a pressing surface that presses, in the distal end direction, an outer edge surface of the medical sheet placed on the second support surface.

(6) In the transfer instrument according to any one of the above (1) to (5), the movement portion may include a liquid supply flow path through which a liquid is supplied to the medical sheet placed on the second support surface.

(7) The transfer instrument according to the above (2) may further include an outer cylinder through which the first shaft is inserted, in which the first support portion and the second support portion may be each flexible and formed in a sheet shape, the first support portion may be formed to be wider than an inner diameter of the outer cylinder, the first support portion, the second support portion, and the medical sheet may be retracted into the outer cylinder with the first support portion, the second support portion, and the medical sheet undergoing curved deformation when the first shaft and the second shaft are moved in a proximal end direction relative to the outer cylinder, and the first support portion, the second support portion, and the medical sheet retracted in the outer cylinder may be unfolded when the first shaft and the second shaft are moved in the distal end direction relative to the outer cylinder to be exposed from the outer cylinder.

(8) In the transfer instrument according to the above (7), the first support portion may include a pair of protrusions protruding upward from both sides of the first support surface in a width direction.

(9) In the transfer instrument according to the above (8), the movement portion may be provided with a retaining slit into which the pair of protrusions are inserted in a retracted state where the first support portion, the second support portion, and the medical sheet are retracted in the outer cylinder.

(10) The transfer instrument according to any one of the above (1) to (9) may further include an endoscope that captures an image of the first support portion, the second support portion, and the medical sheet.

### Brief Description of Drawings

Fig. 1 is a perspective view of a transfer instrument according to an embodiment of the present invention.
Fig. 2 is an exploded perspective view of the transfer instrument illustrated in Fig. 1.
Fig. 3 is a plan view of a distal portion of the transfer instrument illustrated in Fig. 1.
Fig. 4 is a longitudinal cross-sectional view taken along line IV-IV in Fig. 3.
Fig. 5 is a transverse cross-sectional view taken along line V-V in Fig. 3.
Fig. 6 is a flowchart illustrating a procedure of a transfer method for transferring a medical sheet using the transfer instrument illustrated in Fig. 1.
Fig. 7 is a first explanatory diagram of a sheet placing process.
Fig. 8 is a second explanatory diagram of the sheet placing process.
Fig. 9 is an explanatory diagram of a retracting process.
Fig. 10 is a transverse cross-sectional view taken along line X-X in Fig. 9.
Fig. 11 is a transverse cross-sectional view taken along line XI-XI in Fig. 9.
Fig. 12 is an explanatory diagram of a positioning process.
Fig. 13 is an explanatory diagram of an unfolding process.
Fig. 14 is an explanatory diagram of a moving process.
Fig. 15 is an explanatory diagram of a withdrawing process.
Fig. 16A is a partially omitted schematic view of a transfer instrument including an instrument body according to a first modification. Fig. 16B is a transverse cross-sectional view taken along line XVIB-XVIB in Fig. 16A.
Fig. 17 is an explanatory diagram when a second carrier member illustrated in Fig. 16A is at a second position.
Fig. 18 is a perspective view of a transfer instrument including an instrument body according to a second modification.
Fig. 19 is a perspective view of a second carrier member illustrated in Fig. 18 when the second carrier member is at the second position.
Fig. 20 is a longitudinal cross-sectional view of a distal portion of a transfer instrument including an instrument body according to a third modification.
Fig. 21 is a perspective view of a transfer instrument including an instrument body according to a fourth modification.
Fig. 22 is a transverse cross-sectional view of the transfer instrument illustrated in Fig. 21 with a first support portion and a second support portion retracted in an outer cylinder.
Fig. 23 is a partially omitted longitudinal cross-sectional view of a transfer instrument including an instrument body according to a fifth modification.

### Description of Embodiments

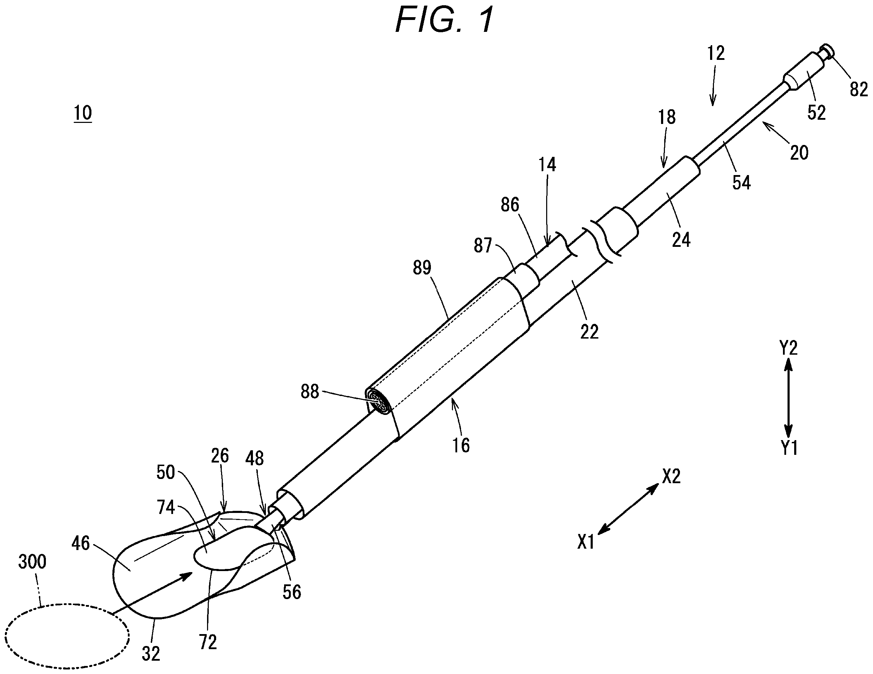

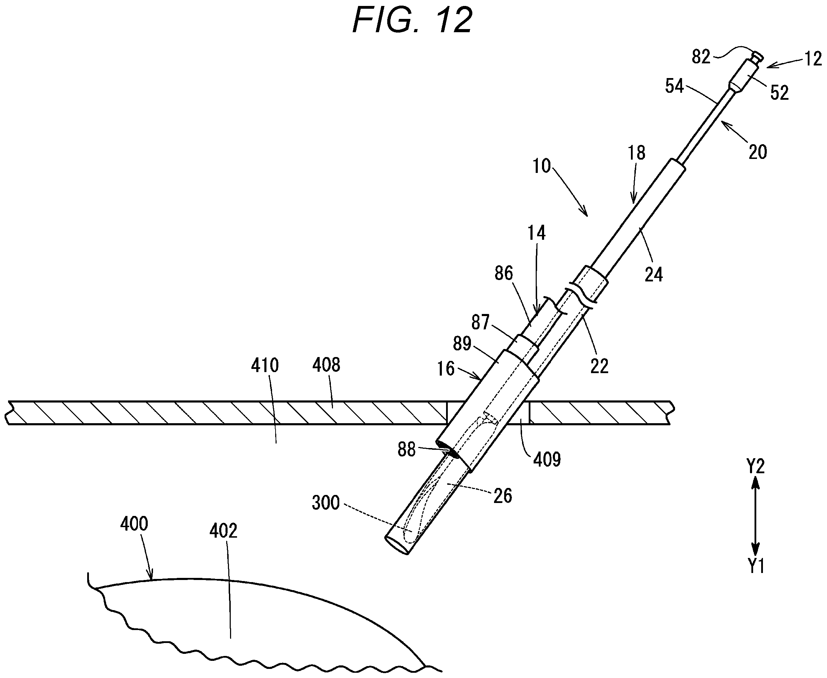

As illustrated in Fig. 1, a transfer instrument 10 according to an embodiment of the present invention is a medical instrument for transferring a medical sheet 300 to a treatment site of a living body. The transfer instrument 10 is used for, for example, the treatment of severe heart failure caused by ischemic heart disease. In this case, the medical sheet 300 is transplanted to a recipient site 402 of a heart 400 (the treatment site of the living body) (see Figs. 12 to 15). The transfer instrument 10 is capable of attaching a plurality of the medical sheets 300 to the recipient site 402.

Examples of such a medical sheet 300 include pharmaceutical products or regenerative medicine products for medical use, a medical instrument, and the like. The medical sheet 300 is formed in a sheet shape such as a film shape or a membrane shape (high viscosity object). Fibrin or the like may be applied to the medical sheet 300 for reinforcement. Examples of the regenerative medicine products including cells include a cell sheet (sheet-shaped cell culture), a spheroid (multicellular spheroid ), and the like. It is possible to form the cell sheet by culturing autologous cells or allogenic cells. The cells constituting the cell sheet include, for example, somatic stem cells (adult stem cells), mesenchymal stem cells, or iPS cells (induced pluripotent stem cells)-derived cardiomyocytes. Examples of the somatic stem cells preferably include skeletal myoblast cells (myoblast cells).

The medical sheet 300 may contain a tissue adhesive, a local anesthetic, or the like. The medical sheet 300 has a thickness of, for example, about 100 µm, and has a diameter of, for example, about 60 mm. Note that the thickness and the diameter (size) of the medical sheet 300 can be set as desired.

The medical sheet 300 may be a sheet to be transplanted to an organ (for example, lung, liver, pancreas, kidney, small intestine, esophagus, or the like) other than the heart 400. Further, the medical sheet 300 may be, for example, an anti-adhesion sheet as long as the sheet is for medical use.

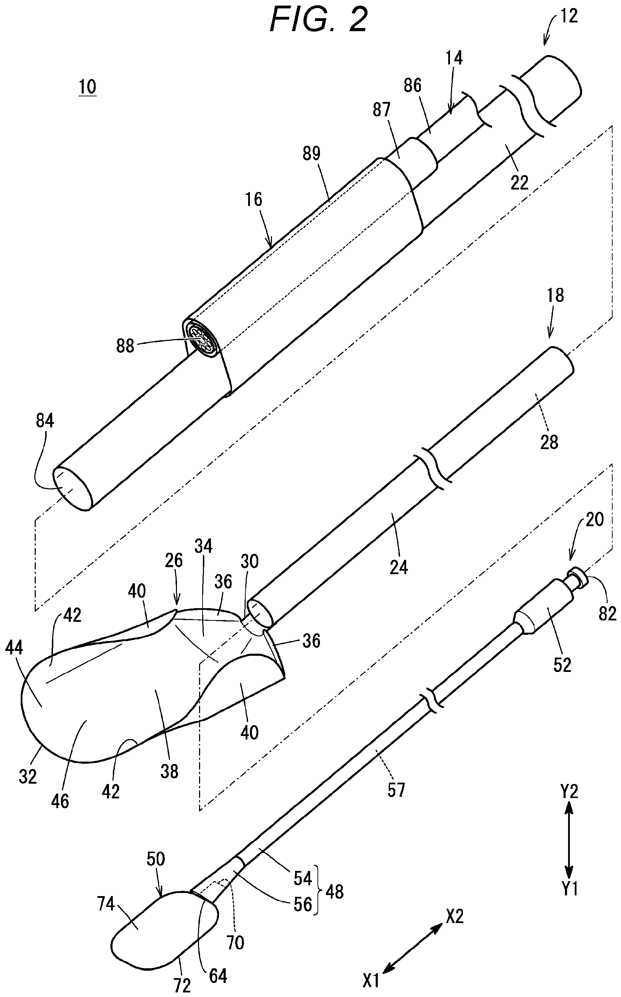

As illustrated in Figs. 1 and 2, the transfer instrument 10 includes an instrument body 12, an endoscope 14, and a fixing member 16. The instrument body 12 includes a first carrier member 18, a second carrier member 20, and an outer cylinder 22.

In Fig. 2, the first carrier member 18 includes a first shaft 24 and a first support portion 26. The first shaft 24 is a circular tube member having a first lumen 28. The first lumen 28 opens at a distal end (end in a direction of arrow X1) of the first shaft 24 and opens at a proximal end (end in a direction of arrow X2) of the first shaft 24.

The first shaft 24 includes, for example, a resin material. Examples of the constituent material of the first shaft 24 include, but not particularly limited to, polyethylene, polypropylene, fluororesin, polyethylene terephthalate, polymethyl methacrylate, polyamide resin, polystyrene, polycarbonate, polyimide, polyetherimide, polyetheretherketone, polyvinyl chloride, and ABS resin. The first shaft 24 may include a metal material.

The first shaft 24 may be flexible. The first shaft 24 may include a flexible tube portion capable of retaining a bent shape. In this case, the first shaft 24 can be bent into an any desired shape in a body cavity and can retaining the bent shape. The flexible tube portion is formed by, for example, forming a tube wall portion in a pleated shape. Note that the flexible tube portion may include a material capable of retaining a bent shape. Specifically, the flexible tube portion can be formed, for example, by inserting a linear metal member capable of retaining a bent shape into the lumen of the first shaft 24 including resin. The flexible tube portion forms only a part of the first shaft 24 in a longitudinal direction. Note that the flexible tube portion may form the entire first shaft 24.

As illustrated in Figs. 2 to 4, the first support portion 26 is attached to a distal portion of the first shaft 24. The first support portion 26 includes, for example, a resin material. The constituent material of the first support portion 26 preferably has transparency, and examples of the constituent material include, but not particularly limited to, polycarbonate, polyamide, polystyrene, polypropylene, polyacetal resin, polyimide, polyetheretherketone, polyethylene terephthalate, and fluororesin.

The first support portion 26 is flexible. A resin sheet member (film member) is bent into a predetermined shape to form the first support portion 26. Alternatively, the sheet member is shaped into a predetermined form by a sheet forming die to form the first support portion 26. It is preferable that the sheet member have, but not particularly limited to, a thickness of, for example, 100 µm or more and 200 µm or less. The first support portion 26 includes a first joint 30 and a first support body 32.

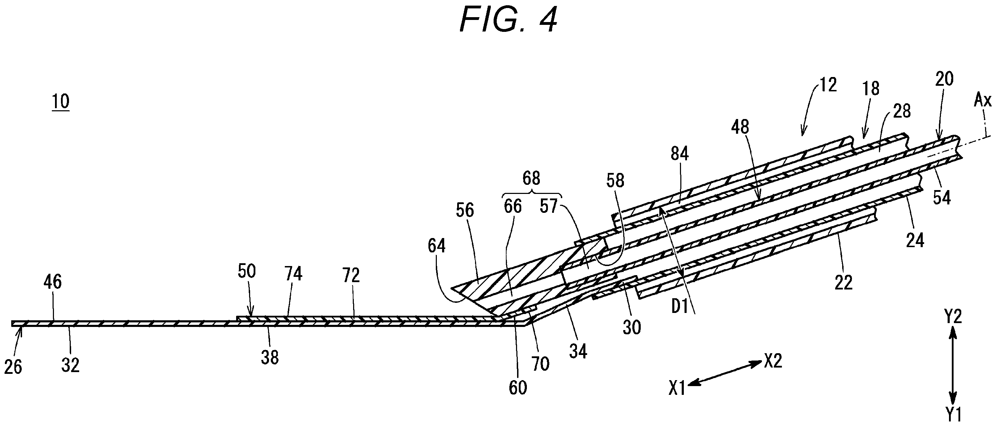

In Fig. 4, the first joint 30 is bonded to an inner peripheral surface of the distal portion of the first shaft 24 with an adhesive. Examples of the adhesive include, but not particularly limited to, a UV adhesive and an instant adhesive (for example, cyanoacrylate-based instant adhesive). The first joint 30 may be thermally fused to the inner peripheral surface of the first shaft 24. In a case where the first joint 30 is joined to the inner peripheral surface of the distal portion of the first shaft 24, the first joint 30 does not form a step on an outer peripheral surface of the first shaft 24, so that the first shaft 24 can be smoothly inserted into the outer cylinder 22. The first joint 30 may be joined to a lower surface (an outer surface facing the direction of arrow Y1) of the distal portion of the first shaft 24.

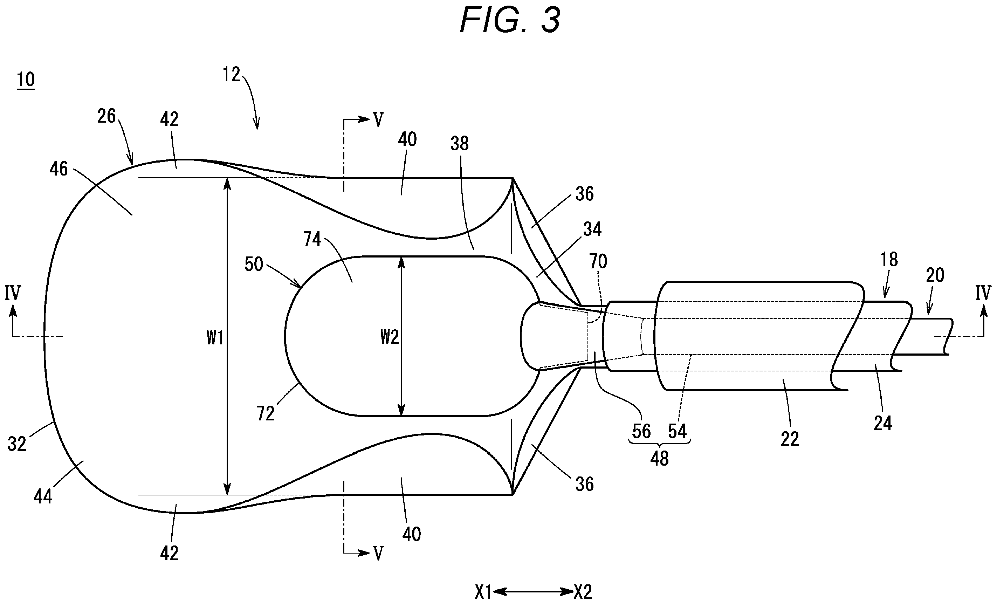

As illustrated in Fig. 3, the first support body 32 extends in a distal end direction from the first joint 30 (see Fig. 4). The first support body 32 includes a proximal-end support portion 34, a pair of first protrusions 36, an intermediate support portion 38, a pair of second protrusions 40, a pair of third protrusions 42, and a distal-end support portion 44.

The proximal-end support portion 34 extends roughly along an axis Ax of the first shaft 24 from a distal end of the first joint 30 in the distal end direction (see Fig. 4). The proximal-end support portion 34 is formed to be wider in its extending direction. In other words, both sides of the proximal-end support portion 34 in a width direction are tapered toward the first joint 30.

In Figs. 2 and 3, the pair of first protrusions 36 protrude upward (in a direction of arrow Y2) from both the sides of the proximal-end support portion 34. The intermediate support portion 38 extends from a distal end of the proximal-end support portion 34 in the distal end direction at an angle relative to the proximal-end support portion 34 so as to approach the axis Ax of the first shaft 24 (see Fig. 4). The intermediate support portion 38 is formed with a substantially constant width over the entire length. Note that the intermediate support portion 38 may be formed to be wider or narrower in the distal end direction.

The pair of second protrusions 40 are connected to distal ends of the pair of first protrusions 36. The pair of second protrusions 40 protrude upward from both sides of the intermediate support portion 38 in the width direction and inward in the width direction of the intermediate support portion 38. The pair of third protrusions 42 are connected to distal ends of the pair of second protrusions 40. The pair of third protrusions 42 protrude upward from both the sides of the intermediate support portion 38 in the width direction and outward in the width direction of the intermediate support portion 38.

The distal-end support portion 44 is connected to a distal end of the intermediate support portion 38 and distal ends of the pair of third protrusions 42. The distal-end support portion 44 protrudes in an arc shape in the distal end direction. In other words, the distal-end support portion 44 is formed to be narrower in the distal end direction.

The first support body 32 includes a first support surface 46 facing upward. Specifically, the first support surface 46 is formed by an upper surface of the intermediate support portion 38, an upper surface of the distal-end support portion 44, and upper surfaces of the pair of third protrusions 42. A lubricant may be applied to the first support surface 46 so as to allow a second support portion 50 (to be described later) of the second carrier member 20 to smoothly slide on the first support surface 46.

A part of the first support surface 46 formed by the upper surface of the intermediate support portion 38 and the upper surface of the distal-end support portion 44 is a flat surface (see Fig. 4). Note that the part of the first support surface 46 formed by the upper surface of the intermediate support portion 38 and the upper surface of the distal-end support portion 44 may be a curved surface that curves in a direction opposite to the direction that the first support surface 46 faces.

As illustrated in Fig. 2, the second carrier member 20 includes a movement portion 48, the second support portion 50, and a connector 52. The movement portion 48 includes a second shaft 54 and a pressing portion 56. The second shaft 54 is a circular tube member having a second lumen 57. The second lumen 57 opens at a distal end (end in the direction of arrow X1) of the second shaft 54 and opens at a proximal end (end in the direction of arrow X2) of the second shaft 54.

The second shaft 54 is longer in the axial direction than the first shaft 24. The second shaft 54 is inserted into the first lumen 28 of the first shaft 24 (see Figs. 1 and 4). In other words, a distal portion of the second shaft 54 protrudes in the distal end direction relative to a distal-end opening of the first shaft 24. A proximal portion of the second shaft 54 protrudes in the proximal end direction relative to a proximal-end opening of the first shaft 24 (see Fig. 1).

The second shaft 54 is configured to follow the shape (angle) of the first support portion 26. As the constituent material of the second shaft 54, for example, a material more flexible than the constituent material of the first shaft 24 is selected. Specifically, examples of the constituent material of the second shaft 54 include a polyamide elastomer, a polyester elastomer, a polyurethane elastomer, polyvinyl chloride, polybutadiene, a silicone rubber, and a metal coil (including a composite with a resin). The second shaft 54 is flexible. Note that in a case where the first shaft 24 includes the flexible tube portion, the second shaft 54 bends along the bent shape of the first shaft 24. The pressing portion 56 is attached to the distal portion of the second shaft 54.

As illustrated in Fig. 4, an insertion hole 58, an attachment surface 60, a pressing surface 64, and a supply hole 66 are formed in the pressing portion 56. The distal portion of the second shaft 54 is inserted into the insertion hole 58. The distal portion of the second shaft 54 is thermally fused to an inner surface forming the insertion hole 58. Note that the distal portion of the second shaft 54 may be joined to the inner surface forming the insertion hole 58 with an adhesive. In Fig. 3, the pressing portion 56 is formed to be wider in the distal end direction when viewed from above. The pressing portion 56 is formed in a trapezoidal shape when viewed from above.

In Fig. 4, the attachment surface 60 is provided on a lower surface of the pressing portion 56. The second support portion 50 is attached to the attachment surface 60. The pressing surface 64 is provided on a distal end surface of the pressing portion 56. The pressing surface 64 presses an outer edge surface of the medical sheet 300 in the distal end direction. The pressing surface 64 extends upward with an inclination in the distal end direction (the direction of arrow X1). The pressing surface 64 includes a flat surface.

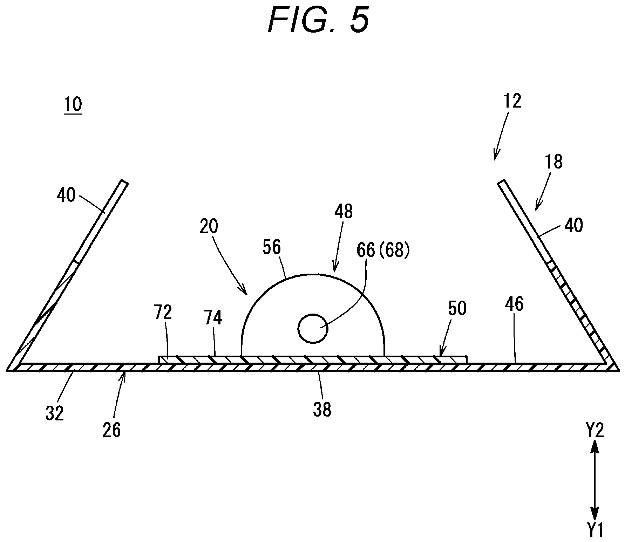

As illustrated in Figs. 4 and 5, the supply hole 66 communicates with the second lumen 57 of the second shaft 54 and opens to the pressing surface 64. The supply hole 66 and the second lumen 57 communicate with each other to form a liquid supply flow path 68. The liquid supply flow path 68 guides, toward a distal end of the pressing portion 56, a liquid for preventing the medical sheet 300 from drying out. As the liquid, for example, a physiological saline solution is used.

In Figs. 2 to 4, the second support portion 50 is formed in a sheet shape. A through hole (hole) may be formed in the second support portion 50. Further, the second support portion 50 may be formed in an annular shape or a mesh shape. The second support portion 50 is flexible. The second support portion 50 includes a second joint 70 and a second support body 72. The second joint 70 is thermally fused to the attachment surface 60 of the pressing portion 56. The second joint 70 may be joined to the attachment surface 60 of the pressing portion 56 by a suitable joining method other than thermal fusion.

The second support body 72 extends in the distal end direction from the second joint 70. The second support body 72 extending from the second joint 70 is shorter than the first support body 32 extending from the first joint 30. The second support body 72 is provided with, on its upper surface, a second support surface 74 on which the medical sheet 300 is placed. The second support surface 74 includes a flat surface. The second support body 72 is smaller than the first support body 32. That is, the second support surface 74 is smaller in area than the first support surface 46. A width W2 of an intermediate portion of the second support body 72 is smaller than a width W1 of the intermediate support portion 38 of the first support body 32.

As illustrated in Fig. 3, a proximal portion of the second support body 72 is formed to be narrower in the proximal end direction. The intermediate portion of the second support body 72 extends with a substantially constant width. The intermediate portion of the second support body 72 may be formed to be wider or narrower in the distal end direction. A distal portion of the second support portion 50 protrudes in an arc shape in the distal end direction. In other words, the second support body 72 is formed to be narrower in the distal end direction. A lubricant may be applied to both surfaces (lower surface and upper surface) of the second support body 72.

In Fig. 2, the connector 52 is attached to the proximal portion of the second shaft 54. The connector 52 is configured as a so-called straight connector. The connector 52 includes a connection port 82 to which a liquid supply instrument (not illustrated) is detachably attached.

In Figs. 1 and 2, the outer cylinder 22 is a cylindrical member having a lumen 84. The lumen 84 opens at a distal end (end in the direction of arrow X1) of the outer cylinder 22 and opens at a proximal end (end in the direction of arrow X2) of the outer cylinder 22. The outer cylinder 22 is flexible. Examples of the constituent material of the outer cylinder 22 are the same as the examples of the constituent material of the first shaft 24 described above. Note that in a case where the first shaft 24 includes the flexible tube portion, the outer cylinder 22 bends along the bent shape of the first shaft 24. Further, in the present embodiment, the outer cylinder 22 may include the flexible tube portion as described above. In this case, the first shaft 24 need not include the flexible tube portion.

The first shaft 24 is inserted into the lumen 84 of the outer cylinder 22. The outer cylinder 22 is shorter in the axial direction than the first shaft 24. In Figs. 3 and 4, an inner diameter D1 of the outer cylinder 22 is smaller than the width W1 of the intermediate support portion 38. The width W1 of the intermediate support portion 38 is substantially identical to a circumferential length of an inner surface of the outer cylinder 22 so as to allow the first support portion 26 to be retracted into the outer cylinder 22 with the first support portion 26 curled into a cylinder along a circumferential direction of an inner peripheral surface of the outer cylinder 22. Note that the width W1 may be shorter or longer than the circumferential length of the inner surface of the outer cylinder 22 as long as the first support portion 26 can be retracted into the outer cylinder 22. The inner diameter D1 of the outer cylinder 22 is smaller than the width W2 of the intermediate portion of the second support body 72.

In Figs. 2 and 4, a distal end surface of the outer cylinder 22 extends in a direction orthogonal to the axial direction of the outer cylinder 22. Note that the distal end surface of the outer cylinder 22 may extend upward with an inclination in the proximal end direction. In this case, for example, in thoracoscopic surgery, the outer cylinder 22 is easily inserted into an incision 409 (Fig. 12) of a chest 408. Further, the angle of the distal end surface of the outer cylinder 22 serves as a guide for a surgeon to recognize the upper surface of the first support portion 26.

As illustrated in Figs. 1 and 2, the endoscope 14 includes a long endoscope main body 86. A distal portion of the endoscope main body 86 is fixed to an outer peripheral surface of the outer cylinder 22 by the fixing member 16. An objective lens 88 provided on a distal end surface of the endoscope main body 86 is oriented toward the distal end of the outer cylinder 22 (in the direction of arrow X1). The distal portion of the endoscope main body 86 is fixed to an intermediate portion of the outer cylinder 22 in the axial direction. Note that the distal portion of the endoscope main body 86 may be fixed to a distal portion of the outer cylinder 22.

The fixing member 16 includes, for example, a fixing cylinder 87 and a fixing tube 89. The fixing cylinder 87 includes, for example, a hard resin material. The endoscope main body 86 can be inserted into a lumen of the fixing cylinder 87. The fixing cylinder 87 is disposed along a longitudinal direction of the outer cylinder 22. The fixing tube 89 is a tube for fixing the fixing cylinder 87 at a predetermined position of the outer cylinder 22. The fixing tube 89 is, for example, a heat-shrinkable tube. Note that the outer cylinder 22 and the fixing cylinder 87 may be integrally molded. Note that how to fix the distal portion of the endoscope main body 86 to the outer cylinder 22 may be determined as desired.

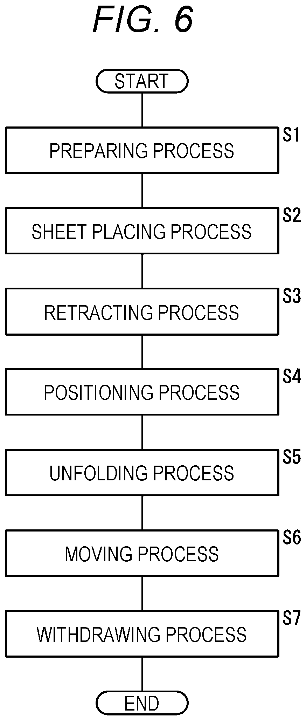

Next, a transfer method for transferring the medical sheet 300 to a treatment site of a living body will be described. Specifically, as illustrated in Figs. 12 to 15, the transfer method for transferring the medical sheet 300 to the recipient site 402 of the heart 400 (the treatment site of the living body) during thoracoscopic surgery will be described. As illustrated in Fig. 6, the transfer method according to the present embodiment includes a preparing process, a sheet placing process, a retracting process, a positioning process, an unfolding process, a moving process, and a withdrawing process.

First, in the preparing process (step S1), the transfer instrument 10 according to the present embodiment described above is prepared. The transfer instrument 10 is brought into an initial state as illustrated in Fig. 1. That is, the first support portion 26 and the second support portion 50 are caused to protrude in the distal end direction from a distal-end opening of the outer cylinder 22, and the second support portion 50 is positioned on the first support surface 46. A proximal portion of the pressing portion 56 is in the first lumen 28 of the first shaft 24.

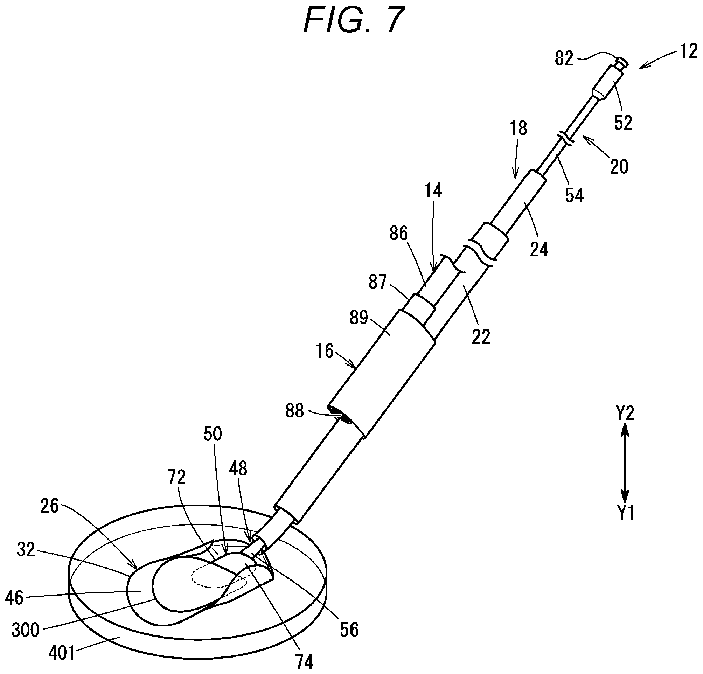

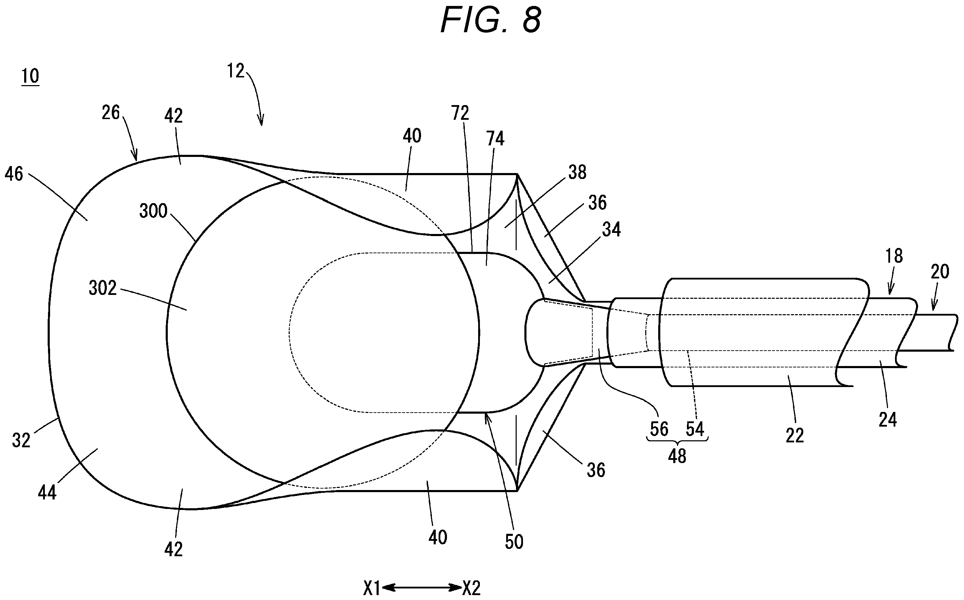

Next, in the sheet placing process (step S2), as illustrated in Fig. 7, the medical sheet 300 cultured in a Petri dish 401 is placed on the second support surface 74. Note that, as illustrated in Fig. 8, the medical sheet 300 sticks out over the second support portion 50 with the medical sheet 300 placed on the second support surface 74. The first support surface 46 supports an overhanging portion 302 of the medical sheet 300 that sticks out over the second support portion 50. The pair of second protrusions 40 prevent the medical sheet 300 from moving (becoming misaligned) in the width direction of the intermediate support portion 38 with the medical sheet 300 placed on the second support surface 74.

Subsequently, in the retracting process (step S3 in Fig. 6), the medical sheet 300 is retracted into the outer cylinder 22 together with the first support portion 26 and the second support portion 50. Specifically, the first shaft 24 of the first carrier member 18 and the second shaft 54 of the second carrier member 20 are moved together in the proximal end direction relative to the outer cylinder 22.

Accordingly, the proximal-end support portion 34 is pulled in the proximal end direction through the distal-end opening of the outer cylinder 22. At this time, when both the tapered sides of the proximal-end support portion 34 come into contact with the distal end surface of the outer cylinder 22, a force acts on the proximal-end support portion 34 to cause the proximal-end support portion 34 to curl along the circumferential direction of the outer cylinder 22. Therefore, the proximal-end support portion 34 is smoothly pulled into the outer cylinder 22 while curling.

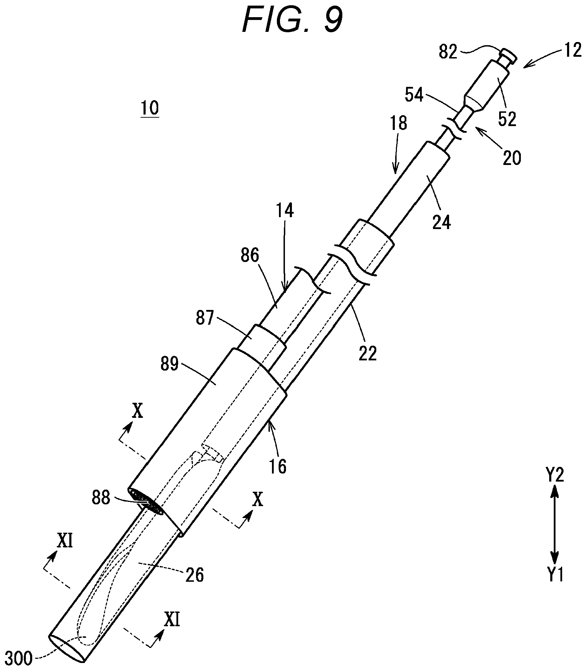

When the proximal-end support portion 34 becomes deformed, a force acts on the intermediate support portion 38 to cause the intermediate support portion 38 to curl along the circumferential direction of the outer cylinder 22, so that the intermediate support portion 38 is pulled into the outer cylinder 22 while curling. At this time, since the width W1 of the intermediate support portion 38 is substantially identical to the circumferential length of the inner surface of the outer cylinder 22, the intermediate support portion 38 is deformed into a cylindrical shape along the inner surface of the outer cylinder 22. The second support body 72 and the medical sheet 300 are inserted into the outer cylinder 22 with the second support body 72 and the medical sheet 300 deformed into a shape corresponding to the shape of the first support body 32. As illustrated in Fig. 9, when the first support portion 26 is entirely inserted into the outer cylinder 22, the retracting process is complete.

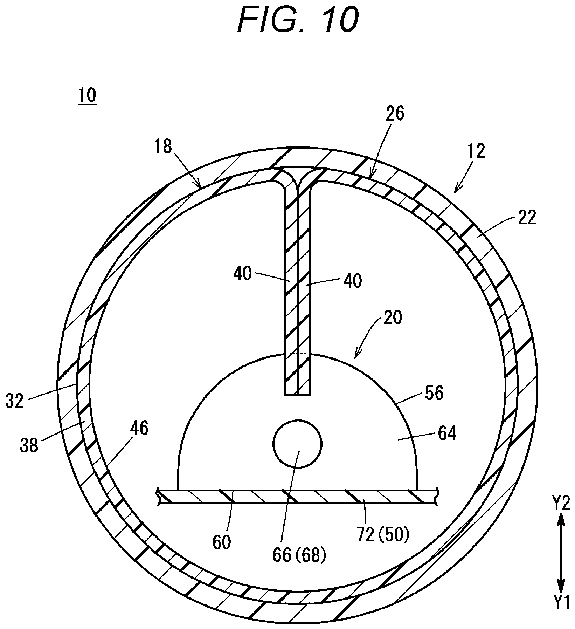

In a retracted state where the first support portion 26, the second support portion 50, and the medical sheet 300 are retracted in the outer cylinder 22, as illustrated in Fig. 10, the pair of second protrusions 40 are positioned in the distal end direction relative to the pressing surface 64 of the pressing portion 56 with the pair of second protrusions 40 in contact with each other. This restricts the movement of the second carrier member 20 in the distal end direction relative to the first carrier member 18. That is, the movement of the second shaft 54 in the distal end direction relative to the first shaft 24 is restricted. Therefore, even in a case where the user pushes by mistake the second shaft 54 in the distal end direction relative to the first shaft 24, it is possible to prevent the second support portion 50 and the medical sheet 300 from protruding from the distal-end opening of the outer cylinder 22.

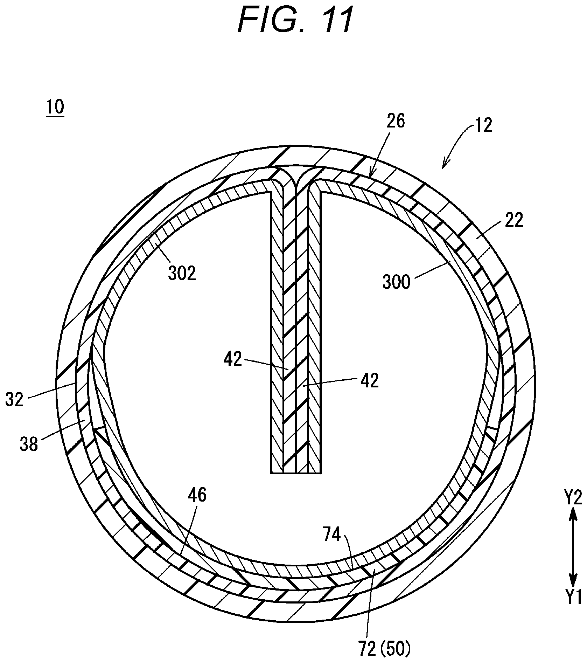

In the retracted state, as illustrated in Fig. 11, the pair of third protrusions 42 extend downward (in the direction of arrow Y1) from an upper end of the outer cylinder 22 with the pair of third protrusions 42 in contact with each other. Protruding end surfaces of the pair of third protrusions 42 are separated from the medical sheet 300. The medical sheet 300 is deformed along the shape of the first support portion 26. That is, this prevents the medical sheet 300 from being folded over in the outer cylinder 22.

Subsequently, in the positioning process (step S4 in Fig. 6), as illustrated in Fig. 12, the transfer instrument 10 is inserted into a chest cavity 410 through the incision 409 of the chest 408. At this time, the distal end of the transfer instrument 10 is positioned near the recipient site 402 of the heart 400, and the distal end of the endoscope 14 is positioned in the chest cavity 410. Note that before the transfer instrument 10 is inserted into the chest cavity 410, a liquid supply instrument (not illustrated) may be connected to the connection port 82 of the connector 52 to introduce a liquid (for example, a physiological saline solution). The liquid introduced from the connection port 82 is guided to the medical sheet 300 through the liquid supply flow path 68 (the second lumen 57 and the supply hole 66). This makes it possible to prevent the medical sheet 300 from drying out.

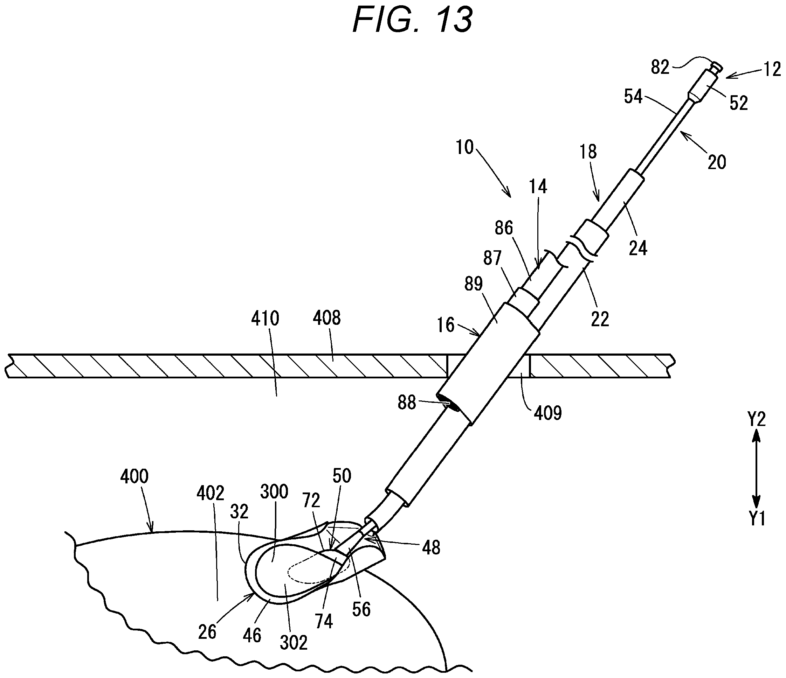

Subsequently, in the unfolding process (step S5 in Fig. 6), as illustrated in Fig. 13, the first support portion 26, the second support portion 50, and the medical sheet 300 are unfolded. Specifically, in the unfolding process, the first shaft 24 and the second shaft 54 are moved in the distal end direction relative to the outer cylinder 22. Accordingly, the first support portion 26 that has moved out of the distal-end opening of the outer cylinder 22 returns to the original shape due to its restoring force. When the first support portion 26 is unfolded, the second support portion 50 and the medical sheet 300 unroll flat.

In the unfolding process, of the second carrier member 20, the second support surface 74 on which the medical sheet 300 is placed is at a first position where the second support surface 74 is entirely positioned on the first support surface 46. At this time, the medical sheet 300 is supported by the first support surface 46 and the second support surface 74. It is therefore possible to prevent the overhanging portion 302 of the medical sheet 300 from becoming crinkled before the medical sheet 300 is transferred to the recipient site 402 of the heart 400.

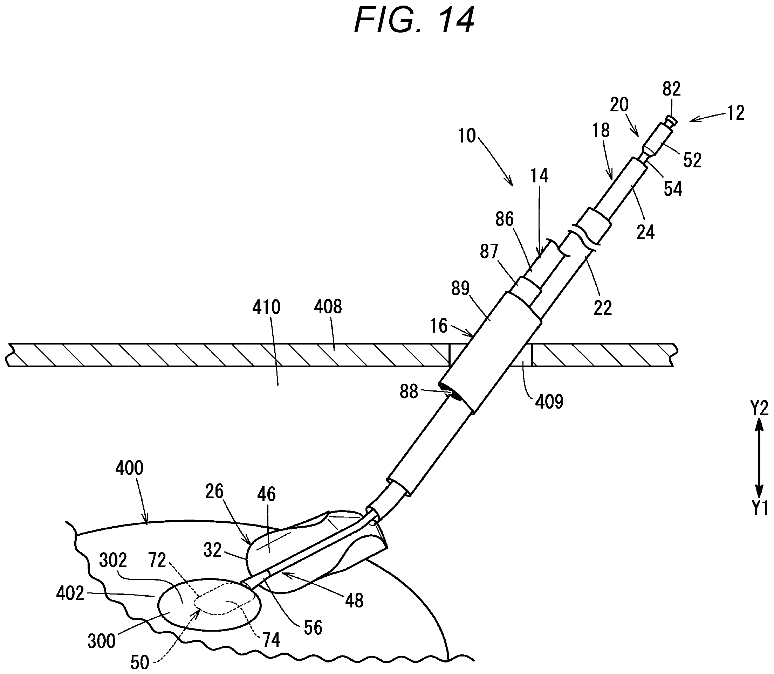

Next, in the moving process (step S6 in Fig. 6), as illustrated in Fig. 14, the second carrier member 20 is moved from the first position to a second position to cause the second support portion 50 on which the medical sheet 300 is placed to protrude in the distal end direction from the distal end of the first support portion 26. Specifically, in the moving process, the second shaft 54 is moved in the distal end direction relative to the first shaft 24.

Accordingly, the second support portion 50 moves in the distal end direction relative to the first support portion 26. At this time, the pressing surface 64 of the pressing portion 56 presses the outer edge surface of the medical sheet 300 in the distal end direction, so that the medical sheet 300 can be smoothly moved over the first support portion 26 even in a case where an area of the overhanging portion 302 of the medical sheet 300 (an area of the medical sheet 300 that is in contact with the first support surface 46) is relatively large. At this time, the medical sheet 300 is entirely positioned in the distal end direction relative to the first support portion 26. In this moving process, the medical sheet 300 is moved to above the recipient site 402 of the heart 400 to bring the overhanging portion 302 of the medical sheet 300 into contact with the recipient site 402.

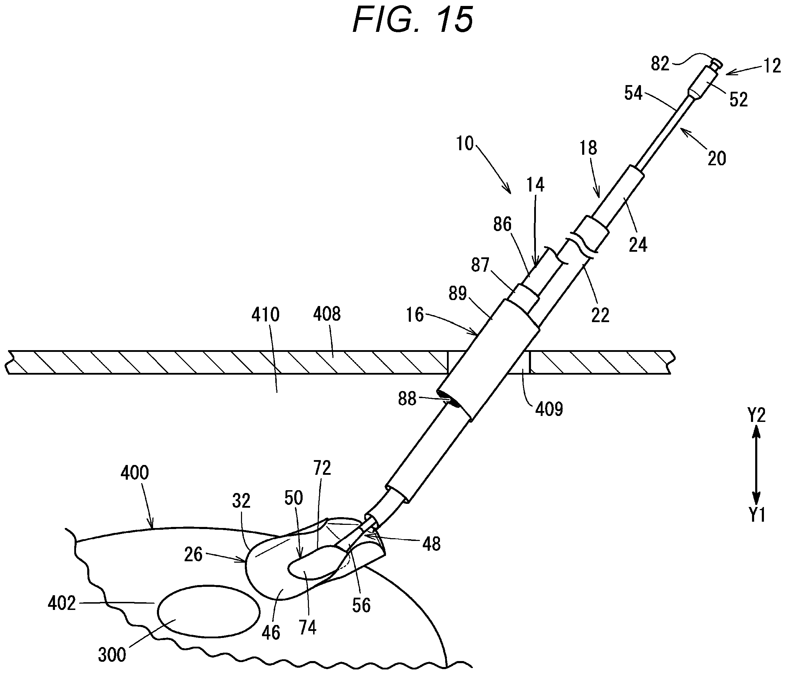

Subsequently, in the withdrawing process (step S7 in Fig. 6), as illustrated in Fig. 15, the second carrier member 20 is moved from the second position to the first position to withdraw the second support portion 50 from between the recipient site 402 and the medical sheet 300. Accordingly, the medical sheet 300 entirely comes into contact with the surface of the recipient site 402. This is the end of the transfer of the medical sheet 300 to the recipient site 402. Subsequently, the transfer instrument 10 is withdrawn from the chest 408 with the first support portion 26 and the second support portion 50 retracted in the outer cylinder 22.

The transfer instrument 10 according to the present embodiment has the following effects.

According to the present embodiment, the medical sheet 300 is supported by the first support surface 46 and the second support surface 74 with the second carrier member 20 at the first position. It is therefore possible to prevent the overhanging portion 302 of the medical sheet 300 that sticks out over the second support surface 74 from becoming crinkled before the medical sheet 300 is transferred to the recipient site 402.

It is further possible to move, by moving the second carrier member 20 from the first position to the second position, the second support portion 50 from the first support portion 26 to the recipient site 402 to place the overhanging portion 302 of the medical sheet 300 on the recipient site 402. Subsequently, it is possible to bring, by withdrawing the second support portion 50 from between the medical sheet 300 and the recipient site 402, the whole of the medical sheet 300 into contact with the recipient site 402. It is therefore possible to efficiently transfer the medical sheet 300 to the recipient site 402.

In this case, since it is not necessary to press the medical sheet 300 with forceps or the like, the medical sheet 300 can be prevented from becoming crinkled. Furthermore, since the second support portion 50 only needs to be withdrawn from between the medical sheet 300 and the recipient site 402 after the second carrier member 20 is moved from the first position to the second position, the medical sheet 300 can be easily transferred to the recipient site 402, and the medical sheet 300 can be prevented from being damaged by forceps.

The first shaft 24 is formed in a tubular shape. The second shaft 54 is inserted into the first lumen 28 of the first shaft 24 with the second shaft 54 movable along the axial direction of the first shaft 24.

Such a configuration can make the transfer instrument 10 compact as compared with a configuration where the second shaft 54 is disposed outside the first shaft 24.

The second support portion 50 is formed in a sheet shape.

Such a configuration can make the step between the first support surface 46 and the second support surface 74 small, so that it is possible to prevent the occurrence of crinkles due to the step.

In a state where the second carrier member 20 is at the second position, the second support surface 74 is entirely positioned in the distal end direction relative to the first support portion 26.

Such a configuration allows the medical sheet 300 to be smoothly transferred to the recipient site 402.

The movement portion 48 includes the pressing surface 64 for pressing the outer edge surface (outer peripheral surface) of the medical sheet 300 placed on the second support surface 74 in the distal end direction.

Such a configuration allows, even in a case where the area of the overhanging portion 302 of the medical sheet 300 (the area of the medical sheet 300 that is in contact with the first support surface 46) is relatively large, the medical sheet 300 to be smoothly moved together with the second support portion 50 in the distal end direction relative to the first support portion 26.

The movement portion 48 includes the liquid supply flow path 68 for supplying a liquid to the medical sheet 300 placed on the second support surface 74.

Such a configuration allows a liquid to be supplied to the medical sheet 300 through the liquid supply flow path 68, so that it is possible to prevent the medical sheet 300 from drying out.

The transfer instrument 10 includes the outer cylinder 22 through which the first shaft 24 is inserted. The first support portion 26 is flexible and formed in a sheet shape. The first support portion 26 and the second support portion 50 are both formed to be wider than the inner diameter D1 of the outer cylinder 22. When the first shaft 24 and the second shaft 54 are moved in the proximal end direction relative to the outer cylinder 22, the first support portion 26, the second support portion 50, and the medical sheet 300 are retracted into the outer cylinder 22 with the first support portion 26, the second support portion 50, and the medical sheet 300 undergoing curved deformation along the circumferential direction of the outer cylinder 22. The first support portion 26, the second support portion 50, and the medical sheet 300 retracted in the outer cylinder 22 are unfolded when the first shaft 24 and the second shaft 54 in the retracted state are moved in the distal end direction relative to the outer cylinder 22 to be exposed from the outer cylinder 22.

Such a configuration allows, in thoracoscopic surgery (body cavity surgery), the outer cylinder 22 to be inserted into the chest cavity 410 (body cavity) from the incision 409 of the chest 408 with the first support portion 26, the second support portion 50, and the medical sheet 300 retracted in the outer cylinder 22. Note that the body cavity includes the chest cavity 410 and the abdominal cavity. Further, in the chest cavity 410, when the first support portion 26 and the second support portion 50 in the retracted state are exposed from the outer cylinder 22, the first support portion 26, the second support portion 50, and the medical sheet 300 can be unfolded. It is therefore possible to make a small incision in the chest 408 of the patient and transfer the medical sheet 300 to the recipient site 402 through the small incision 409, so that it is possible to achieve low invasion as compared with thoracotomy. Furthermore, in thoracoscopic surgery, the medical sheet 300 having a diameter larger than the inner diameter D1 of the outer cylinder 22 can be positioned adjacent to the recipient site 402 (the treatment site of the living body) of the heart 400.

The first support portion 26 includes the pair of second protrusions 40 protruding upward from both sides of the first support surface 46 in the width direction.

Such a configuration allows the pair of second protrusions 40 to prevent the medical sheet 300 from falling outside of the first support body 32.

The transfer instrument 10 further includes the endoscope 14 that captures an image of the first support portion 26, the second support portion 50, and the medical sheet 300.

Such a configuration makes it possible to check, with the endoscope 14, how the medical sheet 300 is transferred to the recipient site 402.

### (First modification)

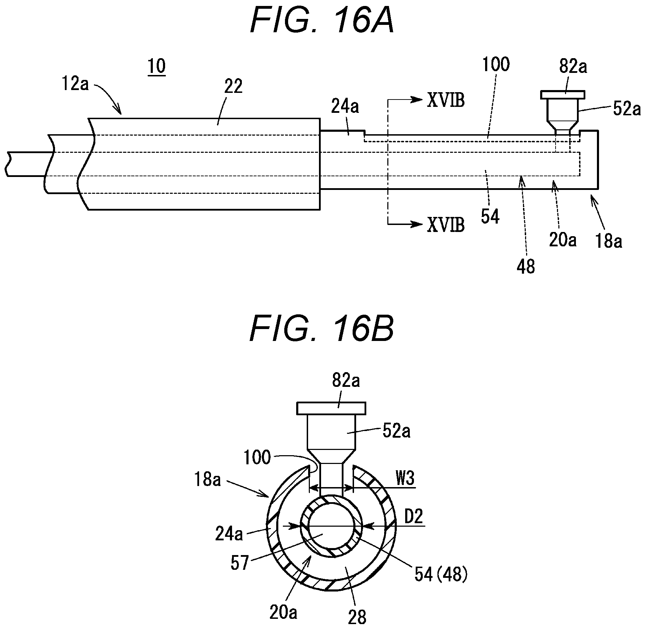

As illustrated in Figs. 16A and 16B, the transfer instrument 10 may include an instrument body 12a according to a first modification. Note that, in the present modification, the same components as those of the above-described instrument body 12 are denoted by the same reference numerals, and the detailed description of the same components will be omitted. The same applies to instrument bodies 12b to 12e according to second to fifth modifications to be described later. The instrument body 12a includes a first carrier member 18a, a second carrier member 20a, and the outer cylinder 22.

The first carrier member 18a includes a first shaft 24a and the first support portion 26 (see Fig. 2). An insertion slit 100 is formed in a proximal portion of the first shaft 24a. The insertion slit 100 communicates with a first lumen 28 of the first shaft 24a. In other words, the insertion slit 100 extends through a peripheral wall of the first shaft 24a in the radial direction. The insertion slit 100 extends along the axial direction of the outer cylinder 22. A width W3 of the insertion slit 100 is smaller than an outer diameter D2 of the second shaft 54 to be described later (see Fig. 16B).

The second carrier member 20a includes the movement portion 48, the second support portion 50 (see Fig. 2), and a connector 52a. The proximal end of the second support portion 50 is sealed so as to prevent the second lumen 57 from communicating with the outside. The connector 52a extends through the insertion slit 100 and extends radially outward from the proximal portion of the second support portion 50. The connector 52a includes a connection port 82a exposed to the outside of the first shaft 24a.

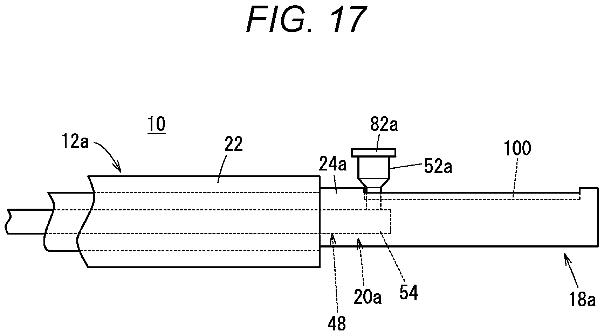

As illustrated in Fig. 17, such a configuration causes the connector 52a to move the insertion slit 100 in the distal end direction when the second carrier member 20a is moved from the first position to the second position (the second support portion 50 is caused to protrude in the distal end direction relative to the first support portion 26). In this case, the overall length of the transfer instrument 10 can be shortened.

### (Second modification)

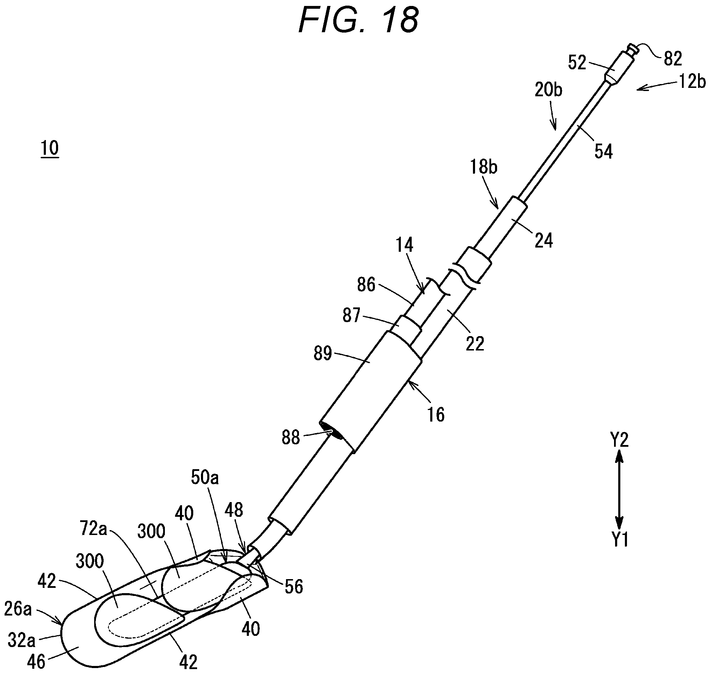

As illustrated in Fig. 18, the transfer instrument 10 may include an instrument body 12b according to a second modification. The instrument body 12b includes a first carrier member 18b, a second carrier member 20b, and the outer cylinder 22.

The first carrier member 18b includes the first shaft 24 and a first support portion 26a. The second carrier member 20b includes the movement portion 48, a second support portion 50a, and the connector 52. A first support body 32a of the first support portion 26a and a second support body 72a of the second support portion 50a are formed to a size that allows a plurality of (in the present embodiment, two) medical sheets 300 to be placed side by side along a direction in which the second support portion 50a moves without overlapping each other.

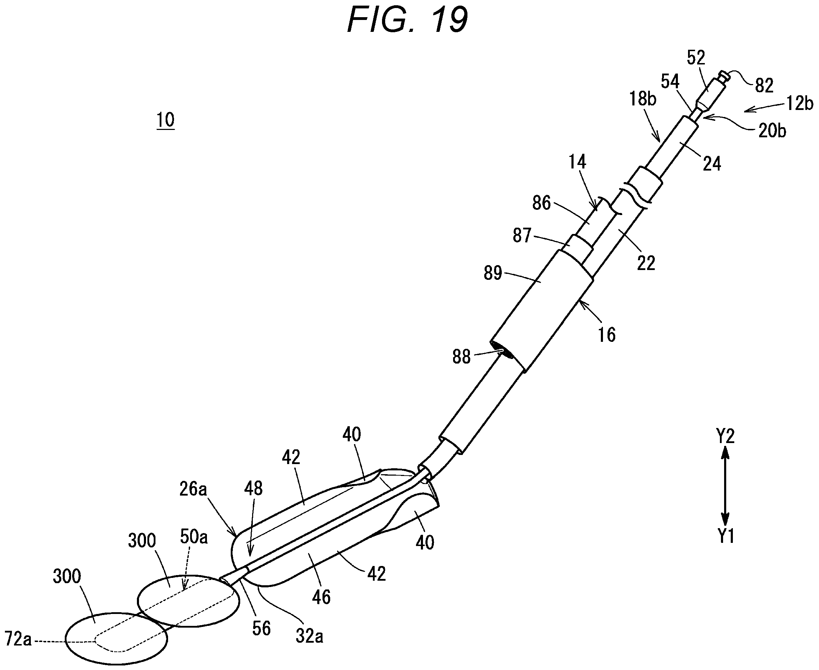

Such a configuration allows, as illustrated in Fig. 19, a plurality of (two) medical sheets 300 to be transferred together to the recipient site 402 of the heart 400 (the treatment site of the living body).

In the present modification, the first support portion 26a and the second support portion 50a may have a size that allows three or more medical sheets 300 to be placed in a row along the direction in which the second support portion 50a moves without overlapping each other.

### (Third modification)

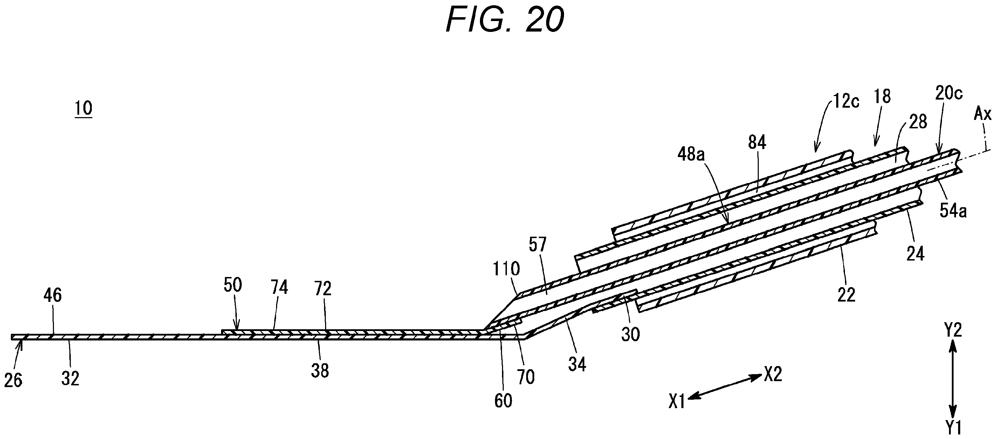

As illustrated in Fig. 20, the transfer instrument 10 may include an instrument body 12c according to a third modification. The instrument body 12c includes the first carrier member 18, a second carrier member 20c, and the outer cylinder 22.

The second carrier member 20c includes a movement portion 48a, the second support portion 50, and the connector 52 (see Fig. 2). The movement portion 48a includes a second shaft 54a. In other words, the movement portion 48a does not include the pressing portion 56 described above.

The second support portion 50 is attached to a distal portion of the second shaft 54a. A distal end surface 110 of the second shaft 54a is an inclined surface extending downward with an inclination in the distal end direction. The distal end surface 110 of the second shaft 54a does not press the outer edge surface of the medical sheet 300 placed on the second support surface 74 in the distal end direction. Even in this case, the medical sheet 300 can be transferred from the first support portion 26 to the recipient site 402 of the heart 400 (the treatment site of the living body) by the second support portion 50.

Such a configuration can make the configuration of the transfer instrument 10 (first carrier member 18) simple.

### (Fourth modification)

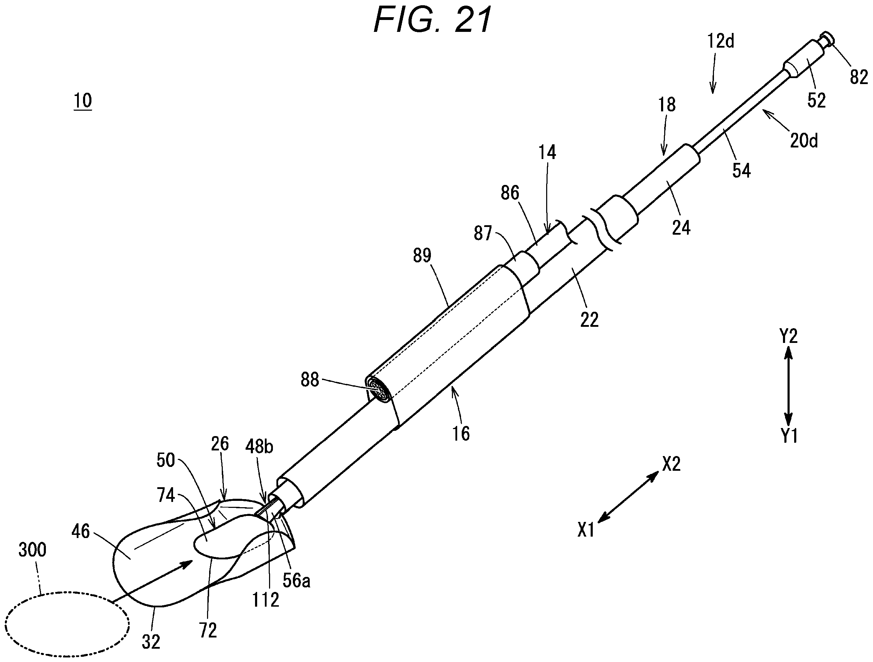

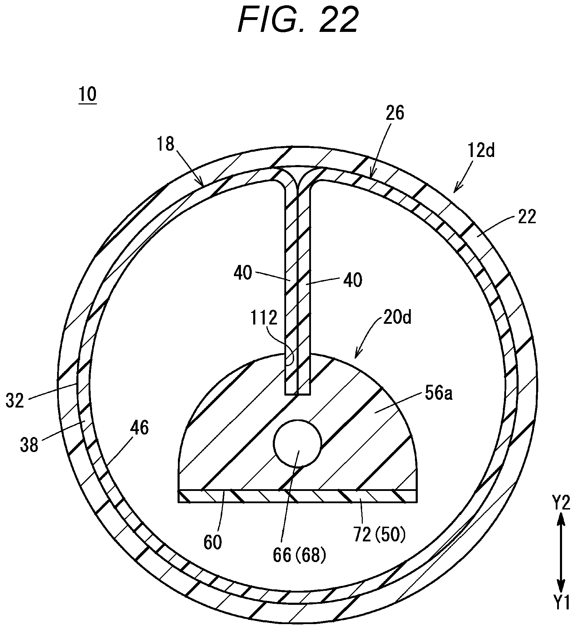

As illustrated in Figs. 21 and 22, the transfer instrument 10 may include an instrument body 12d according to a fourth modification. The instrument body 12d includes the first carrier member 18, a second carrier member 20d, and the outer cylinder 22.

The second carrier member 20d includes a movement portion 48b, the second support portion 50, and the connector 52. The movement portion 48b includes the second shaft 54 and a pressing portion 56a. A retaining slit 112 is formed in an upper surface of the pressing portion 56a. The retaining slit 112 extends over the entire length of the pressing portion 56a. The retaining slit 112 opens upward.

In the present modification, in the retracted state where the first support portion 26, the second support portion 50, and the medical sheet 300 are retracted in the outer cylinder 22, the pair of second protrusions 40 are in the retaining slit 112 of the pressing portion 56a with the pair of second protrusions 40 in contact with each other, as illustrated in Fig. 22. This restricts the movement of the second carrier member 20d relative to the first carrier member 18. That is, the movement of the second shaft 54 along the axial direction relative to the first shaft 24 is restricted. Therefore, even in a case where the user pushes by mistake the second shaft 54 in the distal end direction relative to the first shaft 24, it is possible to prevent the second support portion 50 and the medical sheet 300 from protruding from the distal-end opening of the outer cylinder 22. Further, the second shaft 54 can be prevented from rotating in the circumferential direction relative to the first shaft 24.

The retaining slit 112 into which the pair of second protrusions 40 are inserted in the retracted state is formed in the movement portion 48b.

Such a configuration allows the movement portion 48b to hold the first support portion 26 in the retracted state. It is therefore possible to prevent, even in a case where the second shaft 54 is pushed by mistake in the distal end direction relative to the first shaft 24, the second support portion 50 and the medical sheet 300 from protruding from the outer cylinder 22.

### (Fifth modification)

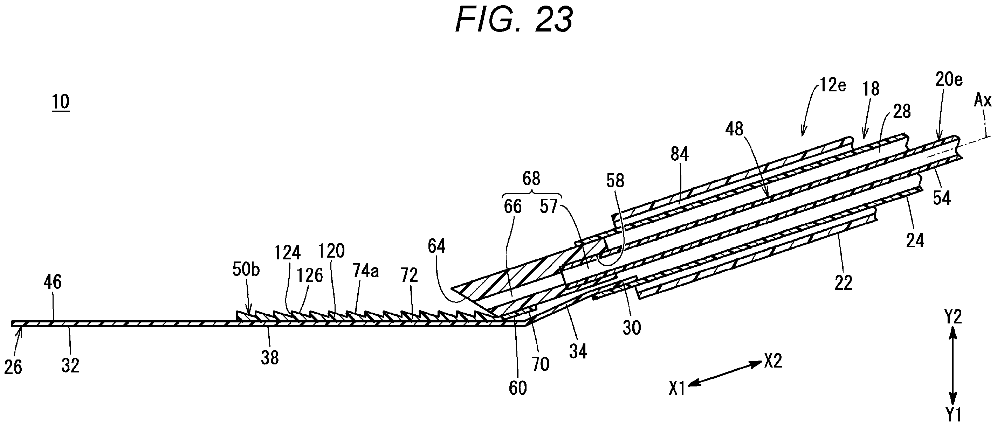

As illustrated in Fig. 23, the transfer instrument 10 may include an instrument body 12e according to a fifth modification. The instrument body 12e includes the first carrier member 18, a second carrier member 20e, and the outer cylinder 22.

The second carrier member 20e includes the movement portion 48, a second support portion 50b, and the connector 52 (see Fig. 1). An upper surface (second support surface 74a) of the second support portion 50b is provided with a plurality of projections 120 protruding upward. Each projection 120 includes a stopper surface 124 and an inclined surface 126. The stopper surface 124 extends along a thickness direction of the second support portion 50b. In other words, the stopper surface 124 faces the distal end direction. The inclined surface 126 extends from a protruding end of the projection 120 with an inclination in the proximal end direction.

Such a configuration allows, when the first support portion 26 is caused to protrude in the distal end direction relative to the second support portion 50b, the stopper surface 124 to prevent the overhanging portion 302 of the medical sheet 300 sticking out over the second support portion 50b from running on to the upper surface (second support surface 74a) of the second support portion 50b. Further, the second support portion 50b can be easily withdrawn from the medical sheet 300 in the withdrawing process.

The transfer instrument 10 according to the present embodiment may have a configuration obtained by combining the configurations according to the first to fifth modifications as desired. The endoscope 14 may be disposed in the lumen 84 of the outer cylinder 22. Further, the endoscope 14 may be provided separately from the instrument bodies 12 and 12a to 12e. Note that the transfer instrument 10 need not include the endoscope 14.

The transfer instrument 10 described above may include a lock mechanism that restricts relative movement of the first shafts 24 and 24a and the second shafts 54 and 54a. The lock mechanism can be switched between a locked state where the relative movement of the first shafts 24 and 24a and the second shafts 54 and 54a along the axial direction of the first shafts 24 and 24a is restricted and a released state where the restriction is removed. In this case, it is possible to move, by bringing the lock mechanism into the locked state, the first shafts 24 and 24a and the second shafts 54 and 54a together relative to the outer cylinder 22 in the retracting process and the positioning process. That is, it is possible to prevent an erroneous operation of moving the second shafts 54 and 54a relative to the first shafts 24 and 24a in the retracting process and the positioning process.

In the transfer instrument 10, in a state where the second carrier members 20, 20a to 20e are at the second position, the second support portions 50, 50a, and 50b need not be entirely positioned in the distal end direction relative to the first support portions 26 and 26a. That is, in the transfer instrument 10, for example, in a state where the second carrier members 20, 20a to 20e are at the second position, the distal ends of the second support bodies 72 and 72a may protrude in the distal end direction relative to the distal ends of the first support portions 26 and 26a, and the proximal portions of the second support bodies 72 and 72a may be positioned on the first support surface 46. Even in this case, the medical sheet 300 can be easily transferred from the first support portions 26 and 26a to the recipient site 402.

Recesses having a shape corresponding to the second support portions 50, 50a, and 50b may be formed on the first support surface 46. Such a configuration can make the step between the first support surface 46 and the second support surface 74 small, so that it is possible to further prevent the medical sheet 300 from becoming crinkled. The second support portions 50, 50a, and 50b may be provided with a marking or recess for indicating a proper position of the medical sheet 300. Further, the second support portions 50, 50a, and 50b may have moderate flexibility to follow a curved recipient site 402.

The transfer instrument 10 may be used for laparoscopic surgery rather than thoracoscopic surgery. Further, the transfer instrument 10 may be used for thoracotomy or laparotomy.

The present embodiment is a sheet transfer method for transferring, with a transfer instrument, a medical sheet to a treatment site of a living body, the transfer instrument including a first carrier member including a first shaft and a first support portion, the first support portion being provided at a distal portion of the first shaft and including a first support surface, and a second carrier member including a movement portion and a second support portion, the movement portion including a second shaft extending along an axial direction of the first shaft, the second support portion being provided at a distal portion of the movement portion and including a second support surface, the sheet transfer method including a preparing process of placing the medical sheet onto the second support surface with the second support surface entirely positioned on the first support surface to support an overhanging portion of the medical sheet, the overhang portion sticking out over the second support surface; a moving process of moving the second shaft in a distal end direction relative to the first shaft to cause the second support surface to protrude in the distal end direction relative to a distal end of the first support portion and bring the overhanging portion into contact with the treatment site after the preparing process; and a withdrawing process of pulling back the second support portion in a proximal end direction to withdraw the second support portion from between the treatment site and the medical sheet after the moving process.

Note that the present invention is not limited to the disclosure, and various configurations can be adopted without departing from the gist of the present invention.

## Claims

1. A transfer instrument that transfers a medical sheet to a treatment site of a living body, the transfer instrument comprising:
a first carrier member including a first shaft and a first support portion, the first support portion being provided at a distal portion of the first shaft and including a first support surface; and
a second carrier member including a movement portion and a second support portion, the movement portion including a second shaft extending along an axial direction of the first shaft, the second support portion being provided at a distal portion of the movement portion and including a second support surface smaller than the first support surface, wherein
the first support surface supports an overhanging portion of the medical sheet, the overhanging portion sticking out over the second support surface with the second support portion positioned on the first support surface, and
the second carrier member is movable relative to the first carrier member from a first position where the second support surface is entirely positioned on the first support surface to a second position where the second support surface is positioned in a distal end direction relative to a distal end of the first support portion.

2. The transfer instrument according to claim 1, wherein
the first shaft is formed in a tubular shape, and
the second shaft is inserted into a lumen of the first shaft so as to be movable along the axial direction of the first shaft.

3. The transfer instrument according to claim 1, wherein
the second support portion is formed in a sheet shape.

4. The transfer instrument according to claim 1, wherein
the second support surface is entirely positioned in the distal end direction relative to the distal end of the first support portion with the second carrier member at the second position.

5. The transfer instrument according to claim 1, wherein
the movement portion includes a pressing surface that presses, in the distal end direction, an outer edge surface of the medical sheet placed on the second support surface.

6. The transfer instrument according to claim 1, wherein
the movement portion includes a liquid supply flow path through which a liquid is supplied to the medical sheet placed on the second support surface.

7. The transfer instrument according to claim 2, further comprising
an outer cylinder through which the first shaft is inserted, wherein
the first support portion and the second support portion are each flexible and formed in a sheet shape,
the first support portion is formed to be wider than an inner diameter of the outer cylinder,
the first support portion, the second support portion, and the medical sheet are retracted into the outer cylinder with the first support portion, the second support portion, and the medical sheet undergoing curved deformation when the first shaft and the second shaft are moved in a proximal end direction relative to the outer cylinder, and
the first support portion, the second support portion, and the medical sheet retracted in the outer cylinder are unfolded when the first shaft and the second shaft are moved in the distal end direction relative to the outer cylinder to be exposed from the outer cylinder.

8. The transfer instrument according to claim 7, wherein
the first support portion includes a pair of protrusions protruding upward from both sides of the first support surface in a width direction.

9. The transfer instrument according to claim 8, wherein
the movement portion is provided with a retaining slit into which the pair of protrusions are inserted in a retracted state where the first support portion, the second support portion, and the medical sheet are retracted in the outer cylinder.

10. The transfer instrument according to any one of claims 1 to 9, further comprising
an endoscope that captures an image of the first support portion, the second support portion, and the medical sheet.
